# EUROPEAN PATENT APPLICATION

(11) **EP 1 880 985 A1**
(43) Date of publication of application: **23.01.2008**
(21) Application number: 06253501.8
(22) Date of filing: 04.07.2006
(51) Int. Cl.: C07C 4/02

(54) **Method for start-up of an autothermal cracking reaction**

(71) Applicant: INEOS EUROPE LIMITED, Lyndhurst, Hampshire, SO43 7FG (GB)
(72) Inventor: The designation of the inventor has not yet been filed
(74) Representative: King, Alex

(57) **Abstract**

The present invention provides a method for starting an autothermal cracking reaction for the production of one or more olefins, said autothermal cracking reaction comprising contacting a paraffinic hydrocarbon and oxygen with a catalyst at an elevated pressure, P1**,** which start-up method comprises:
a) establishing a flow of a purge gas immediately downstream of the catalyst,
b) contacting a pre-heated feed mixture comprising hydrogen,oxygen and a diluent with the catalyst at an elevated pressure, P2, which is less than P1, wherein the hydrogen to oxygen molar ratio is at least 0.5:1 and wherein the total feed mixture comprises less than 20wt% oxygen and at least 50wt% diluent,
c) increasing the oxygen concentration in the feed mixture until a catalyst exit temperature of at least 700°C is reached, and
d) increasing the pressure to the desired reaction pressure P1.

## Description

The present invention relates to process start-up, and, in particular, to the start-up of an autothermal cracking process.

Autothermal cracking is a route to olefins in which a hydrocarbon feed is mixed with oxygen and passed over an autothermal cracking catalyst. Combustion is initiated on the catalyst surface and the heat required to raise the reactants to process temperature and to carry out the endothermic cracking process is generated in situ. Such a process is described for example in EP 332289B; EP-529793B; EP-A-0709446 and WO 00/14035.

The autothermal cracking reaction is started by initiating the combustion of hydrocarbon and/or co-fed hydrogen with oxygen. On combustion a rapid increase occurs in the temperatures of both the catalyst and associated reactor structure, such as any catalyst holder and other areas downstream of the catalyst. Whilst this "temperature shock" may be tolerated at laboratory scale, such that start-up may be initiated in a number of ways, at commercial scale it is desired to minimise any such "temperature shock" due to long term effects on the reactor and the catalyst. For example, for the catalyst itself, a laboratory catalyst may only be used once or twice, and over a relatively short period of time, whereas a commercial catalyst charge would be desired to operate for a much longer period of time. Also, small amounts of dust which may be produced by thermal shock to a catalyst may not cause problems at a laboratory scale, but may cause significant downstream problems at commercial scale, where more downstream processing is likely to be present than at laboratory scale.

Especially at elevated pressure, it is also important that flammable mixtures of hydrocarbon and oxygen are not present downstream of the catalyst. Although it leads to large and rapid temperature increases, at laboratory scale reactants can be introduced at pressure and ignited by a suitable ignition source to ensure initiation of combustion occurs or a feed composition can be used that "guarantees" the initiation of the combustion reaction, typically by ensuring highly reactive mixtures (which will lead to large temperature increases). When it is desired to operate the autothermal cracking reaction at elevated pressures at commercial scale such initiation is not desirable and, therefore, an alternative start-up is required.

It is desired therefore to find a safe method for starting an autothermal cracking reaction at elevated pressure, which can be operated at commercial scale and which provides the minimum amount of thermal shock to the catalyst and reactor.

It has now been found that a particularly advantageous start-up may be achieved by a controlled initiation at a pressure lower than the final desired operating pressure.

Thus, in a first aspect, the present invention provides a method for starting an autothermal cracking reaction for the production of one or more olefins, said autothermal cracking reaction comprising contacting a paraffinic hydrocarbon and oxygen with a catalyst at an elevated pressure, P1, which start-up method comprises:
a) establishing a flow of a purge gas immediately downstream of the catalyst,
b) contacting a pre-heated feed mixture comprising hydrogen,oxygen and a diluent with the catalyst at an elevated pressure, P2, which is less than P1, wherein the hydrogen to oxygen molar ratio is at least 0.5:1 and wherein the total feed mixture comprises less than 20wt% oxygen and at least 50wt% diluent,
c) increasing the oxygen concentration in the feed mixture until a catalyst exit temperature of at least 700°C is reached, and
d) increasing the pressure to the desired reaction pressure P1.

In step (a) a flow of a purge gas is established immediately downstream of the catalyst. This is a critical safety requirement for the method of the present invention and ensures that should any oxygen not be consumed when introduced, for example if the combustion reaction does not occur, then a flammable mixture downstream of the catalyst does not occur. The purge gas acts as a diluent to ensure a non-flammable mixture is obtained, and may be an inert gas, such as nitrogen, or steam or may be hydrocarbon-containing, such as fuel gas or feed gas.

In step (b) initial contact of hydrogen and oxygen with the catalyst is provided. Most preferably, prior to introduction of any oxygen in this step, the catalyst and reactor internals are pre-heated by passing a hot gas, suitably either an inert gas or hydrocarbon, through the reactor and over the catalyst. This may be done for a suitable period of time to ensure heating of the catalyst and reactor internals, typically at least 10 minutes. The hot gas is typically at a temperature of at least 100°C, preferably at least 150°C.

In addition, the feed system used for the introduction of the oxygen is purged with an inert gas to ensure that no contamination occurs with hydrocarbon containing gas.

The pre-heating of the catalyst helps to ensure that initiation of combustion occurs when the oxygen and hydrogen are subsequently contacted with the catalyst in step (b), and also to further minimise the temperature rise on the catalyst and reactor internals when such combustion occurs. Preferably the catalyst is pre-heated to a temperature of at least 150°C, such as 150°C to 250°C, typically 170°C to 200°C.

The diluent to be used in step (b) may also be used for this pre-heating step, optionally with the required hydrogen for step (b), or one or both may only be introduced shortly before the oxygen is introduced.

The diluent acts to provide a heat load in the pre-heated feed mixture, which, in conjunction with the limits on oxygen concentration in the feed mixture limits the temperature reached when the hydrogen is combusted. The actual catalyst exit temperature also depends on the actual concentration of hydrogen and oxygen in the pre-heated feed mixture. Typically, the catalyst exit temperature at this stage will be in the range 400 to 600°C. In contrast, if just hydrogen and oxygen at stoichiometric ratio were used the reaction temperature would rapidly rise to several thousand degrees.

The diluent may be an inert gas, for example nitrogen or steam, but in a preferred embodiment, comprises the paraffinic hydrocarbon to be cracked.

At the temperatures generally reached at this stage essentially no or only limited hydrocarbon combustion will occur (depending on the hydrocarbon to be cracked), and the combustion is controlled by the hydrogen and oxygen so the paraffinic hydrocarbon effectively acts as an inert diluent.

The hydrogen to oxygen molar ratio in step (b) is at least 0.5:1, which ensures that the reaction can be started readily. Preferably, the hydrogen to oxygen molar ratio is at least 1:1, more preferably at least 2:1, to minimise the amount of oxygen, if any, which exits the catalyst.

The advantages of operating at less than reaction pressure in this step include:
1) Less feed mixture is "wasted" during start-up (no olefins are produced at this stage),
2) The feed mixture is less constrained by flammability constraints, allowing higher feed pre-heat to be used giving more reliable initiation of the combustion reaction and less "temperature shock" on the catalyst and reactor internals,
3) The combustion reaction is easier to initiate.

No ignition source is required to initiate reaction in step (b). Preferably, P2 is at least 5 barg, typically 5 to 15 barg. P2 is also dependent on P1, usually at least 5 barg below P1.

Preferably the initial oxygen concentration (oxygen concentration in the total feed mixture of step (b)) is less than 15wt%.

Preferably the initial oxygen concentration is at least 5wt%.

Most the preferably the initial oxygen concentration is in the range 8 to 12 wt%.

In step (c), the oxygen feed concentration is increased until a catalyst exit temperature of at least 700°C is reached. Increasing the oxygen increases the heat generated by combustion, increasing the catalyst exit temperature.

Preferably the oxygen is increased until the catalyst exit temperature is 750°C or above. Preferably the oxygen is increased at a rate such that a relatively slow temperature increase is obtained in the catalyst exit temperature, typically less than 10°C per minute.

Typically, the oxygen concentration in the feed mixture once the desired catalyst exit temperature is reached is in the range 15 to 40wt%, such as 20 to 40 wt%, for example 20 to 30wt%. In the preferred embodiment where the paraffinic hydrocarbon is already present in the pre-heated feed mixture as a diluent, as the temperature increases hydrocarbon combustion will also start to be observed, although the actual temperature at which this occurs will be dependent on the specific paraffinic hydrocarbon. For example, for ethane a temperature of approximately 600°C is required, for propane it is approximately 550°C. As the temperature increases, cracking will also be observed. It is important to maintain the hydrogen to oxygen molar ratio at 0.5:1 or above (preferably at least 1:1 and more preferably at 2:1 or above) at least until combustion of the paraffinic hydrocarbon is observed, to ensure that high oxygen conversion is achieved. However, once hydrocarbon combustion reactions are occurring, the hydrogen need not be maintained at such a molar ratio to oxygen although equally this may still be desired.

Where paraffinic hydrocarbon is not present in the pre-heated feed mixture it may be added during this stage (stage (c)) either before or after the desired catalyst exit temperature is reached. For example, paraffinic hydrocarbon may be added whilst the oxygen concentration is being increased, in which case combustion of hydrocarbon will also increase as the temperature increases. Alternatively, paraffinic hydrocarbon may be added once the desired catalyst exit temperature is reached in step (c) and prior to step (d) of the present invention. Again, it is important to maintain the hydrogen to oxygen molar ratio at 0.5:1 or above (preferably at least 1:1 and more preferably at 2:1 or above) whilst the oxygen concentration is increased and at least until (introduction and) combustion of paraffinic hydrocarbon is observed, to ensure that high oxygen conversion is achieved. As before, once hydrocarbon combustion reactions are occurring, the hydrogen need not be maintained at such a molar ratio to oxygen although equally this may still be desired.

Where it has not been previously added, it is also possible to add the paraffinic hydrocarbon during or after step (d). (In this case the hydrogen to oxygen molar ratio is maintained at at least 0.5:1, preferably at least 1:1 and more preferably at 2:1 or above, throughout step (c) and until combustion of paraffinic hydrocarbon is observed in step (d).)

However, it is generally preferred that the paraffinic hydrocarbon to be cracked is present prior to increasing the pressure in step (d), such that a "stable" autothermal cracking reaction is occurring at the pressure P2. In particular, in a further preferred embodiment, the cracking reaction is held after step (c) at the temperature reached in step (c) ("reaction temperature") for a "catalyst activation" period before step (d), typically at least 15 minutes, such as approximately 30 minutes. It has been found that this provides improved subsequent catalyst performance. At this stage it is generally also desired to alter the hydrogen feed rate to that desired for the "final" process if this has not already been done. At this stage, once stable operation has been obtained, it is also generally desired to turn on downstream safety systems, such as those described in WO 02/30856 and (slowly) reduce the flow of purge gas.

In step (d) the pressure is increased to the desired reaction pressure, P1. This should be performed by increasing flow rates with the pressure to maintain face velocity as constant as possible, to minimize changes in "cooling" effect of gas flow rate. P 1 is typically at least 10 barg, usually at least 15 barg.

In the autothermal cracking reaction, combustion of the paraffinic hydrocarbon occurs on the catalyst surface which generates the temperature necessary to carry out the endothermic cracking process to produce olefins. In a preferred embodiment, hydrogen is continued to be co-fed to the reaction even once at the desired pressure P1. Combustion of hydrogen to generate heat reduces the amount of hydrocarbon combustion necessary, improving the selectivity of the process.

The paraffinic hydrocarbon to be cracked in the autothermal cracking reaction may be any suitable paraffinic hydrocarbon. Typically the paraffinic hydrocarbon has at least 2 carbon atoms, and is most preferably one or more of ethane, propane or butanes. It may be substantially pure or may be in admixture with other hydrocarbons. Optionally other materials, for example methane, nitrogen, carbon monoxide, carbon dioxide and steam, may be present in the feed to the autothermal cracking process.

The oxygen is provided in the form of a molecular oxygen-containing gas, suitably either air or (molecular) oxygen.

As noted, although not essential, even when the reaction is operating at the required pressure, P1, it is preferred that hydrogen is fed to the autothermal reaction with the paraffinic hydrocarbon, oxygen and any other feed components. Suitably, the molar ratio of hydrogen to oxygen is in the range 0.1 to 3, preferably, in the range 0.2 to 1.

When the reaction is operating at the required pressure, P1, the hydrocarbon and oxygen may be contacted with the catalyst in any suitable molar ratio provided that the ATC product stream comprising olefins is produced. The preferred stoichiometric ratio of hydrocarbon to oxygen is 5 to 16, preferably, 5 to 13.5 times, preferably, 6 to 10 times the stoichiometric ratio of hydrocarbon to oxygen required for complete combustion of the hydrocarbon to carbon dioxide and water.

If not in this ratio prior to this point, a ratio of hydrocarbon to oxygen within these ranges is preferably obtained by adjusting the relative components during step (c) and/or prior to step (d).

Once the reaction is operating at the required pressure, P1, typically the reactants are passed over the catalyst at a pressure dependent gas hourly space velocity of greater than 10,000 h⁻¹ barg⁻¹, preferably greater than 20,000 h⁻¹ barg⁻¹ and, most preferably, greater than 100,000 h-¹ barg⁻¹. For example, where P1 is 20 barg pressure, the gas hourly space velocity is, most preferably, greater than 2,000,000 h⁻¹.

The catalyst may be any catalyst capable of supporting combustion beyond the fuel rich limit of flammability. The catalyst usually comprises a Group VIII metal as its catalytic component. Suitable Group VIII metals include platinum, palladium, ruthenium, rhodium, osmium and iridium. Rhodium, and more particularly, platinum and palladium are preferred.

Once the reaction is operating at the required pressure, P1, the autothermal cracking reaction may suitably be carried out at a catalyst exit temperature in the range 700°C to 1200°C. Suitably the catalyst exit temperature is at least 720°C such as at least 750°C. Preferably, the autothermal cracking step is carried out at a catalyst exit temperature in the range 800°C to 1050°C and, most preferably, in the range 820°C to 1000°C.

Preferably the autothermal cracking process is operated at a pressure P1 of at least 10barg. Preferably the autothermal cracking process is operated at a pressure of 15barg to 40barg and advantageously of 20barg to 30barg e.g. 25barg.

The product stream is usually quenched as it emerges from the reaction chamber to avoid further reactions taking place and the temperature of the stream is reduced to a temperature between 750-600°C. The quenched product stream may then by passed to the treatment steps for removal of oxygenates, aromatics, carbon dioxide and optionally oxygen as required.

The quench involves introduction of a quench gas or liquid (quenchant) into the product stream, usually at an elevated temperature and pressure, for example steam may be injected at 100 barg and 300°C. It is desired that the quench is operating and hot prior to step (b), and, thus, in a most preferred embodiment of the present invention, the quench is started prior to introduction of oxygen in step (b) of the present invention. Although the quench need not be used to actually quench the product stream until olefins are being produced, when the quench is first turned on it is likely to result in introduction of relatively cold quenchant whilst it comes up to operating temperature, and if the quench metalwork is already at much higher temperatures than this because combustion reactions have started, this can cause stresses on the metalwork. By getting the quench system operating close to its standard operating temperature at the much lower temperatures in the reactor prior to initial combustion, this risk is avoided. The quench need only be operated at relatively low flow rates until olefin production is obtained.

The autothermal cracking reaction typically produces a gaseous product stream comprising one or more olefins, hydrogen, carbon monoxide and carbon dioxide. The one or more olefins typically comprise ethene, propene, butene and higher olefins. In addition, the product stream will usually also comprise alkanes, such as methane, water, dienes, such as butadiene, acetylenes, oxygenates and aromatic compounds, such as naphthalenes and toluene.

The autothermal cracking reaction may be operated such that less than 100% oxygen conversion is obtained, such that unreacted oxygen is present in the product stream. This is typically achieved by controlling the severity of reaction, for example, by control of the hydrocarbon to oxygen ratio and/or the space velocity. Where hydrogen is present in the feed, the severity is most preferably controlled by control of the hydrogen to oxygen ratio.

Operation at less than 100% oxygen conversion has the advantage of mitigating coke formation in the autothermal cracking reaction. Without wishing to be bound by theory, it is believed that this is related to the fact that at least some oxygen is present at all points in the reaction zone.

Where oxygen is present in the product stream it may, if necessary, be removed by contacting with an oxygen removal bed as described in WO 2004/033598.

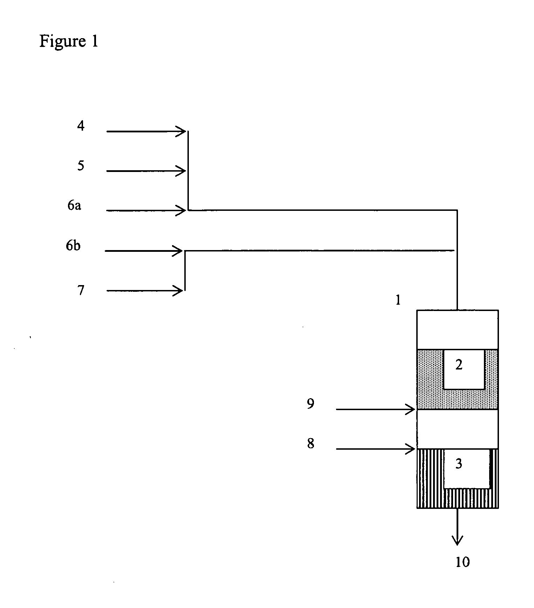

The present invention will now be described with respect to Figure 1 and the following example.

Figure 1 shows in schematic form an autothermal cracking reactor for use at commercial scale. The reactor (1) comprises a catalyst zone (2) and a quench zone (3), is provided with feed lines for hydrocarbon (4), hydrogen (5), nitrogen (6a and 6b) and oxygen (7) upstream of the catalyst zone (2), and for a nitrogen purge (8) downstream of the catalyst zone (2) and quench zone (3).

The upstream feedlines (4-7) can be provided with preheating (not shown).

Steam can be injected to the quench zone (3) via line (9) into the product stream from the autothermal cracking reaction, to give a quenched product stream (10).

### Example 1

A catalyst comprising 3wt% platinum on an alumina foam was placed in an autothermal cracking reactor as shown in Figure 1.

It is desired to perform an autothermal cracking reaction for the cracking of ethane at a pressure (P1) of 20 barg.

Prior to reaction, the reactor and all feed lines are purged with nitrogen (through lines 6a and 6b). The nitrogen purge from line 6b through line 7 (the oxygen system) is maintained at a high flow rate throughout to prevent back-flow of ethane when the oxygen is added. Subsequently, heated ethane and hydrogen are passed from lines 4 and 5 through the catalyst and reactor in order to pre-heat the catalyst and reactor internals to 180°C. The reactor zone (2) is maintained at a pressure of 10 barg. The relative flow rates of ethane and hydrogen are approximately 50:1 (wt:wt).

During this preheating of the reactor and internals, saturated steam at 20 barg and 200°C is introduced to the quench at 10% of normal flow rates in order to start the quench before the reactor internals are heated further.

The ethane flow rate through line (6) is reduced to give an ethane to hydrogen relative flow rate of approximately 30:1 (wt:wt). Subsequently, oxygen is added at a hydrogen to oxygen weight ratio of approximately 5.3:1 in order to start the reaction. These values correspond to an oxygen concentration of 11wt%, hydrogen concentration of 2wt% and ethane concentration of 67wt% (balance nitrogen), and a hydrogen to oxygen molar ratio of 3:1.

Combustion is initiated immediately, and the catalyst exit temperature rises from 180°C to between 500 and 600°C.

The combustion reaction is held under these conditions for 30 minutes in order to allow the reactor internals to heat up.

The oxygen and hydrogen flow rates are then increased over a period of 30 minutes. The new conditions correspond to an oxygen concentration of 25wt%, hydrogen concentration of 2.8wt% and ethane concentration of 56wt% (balance nitrogen), and a hydrogen to oxygen molar ratio of 1.8:1.

During the period of ramping hydrogen and oxygen feeds, the catalyst exit temperature increases to approximately 830°C, ethane combustion starts to occur and ethylene is observed in the product stream.

As soon as the temperature of the reaction has reached 750°C the purge nitrogen is slowly removed and, subsequently, the quench flow rate is increased to normal operating conditions.

Once stable reaction is achieved and the temperature is suitably above the trip settings, hydrogen flow rate is reduced further to a hydrogen to oxygen molar ratio of 1:1, and the reaction is maintained under these conditions for an activation period.

The pressure is then increased to the final desired pressure over a period of several hours, whilst increasing total feed flow rates to maintain the feed composition and the feed face velocity on the catalyst.

## Claims

1. A method for starting an autothermal cracking reaction for the production of one or more olefins, said autothermal cracking reaction comprising contacting a paraffinic hydrocarbon and oxygen with a catalyst at an elevated pressure, P1, which start-up method comprises:
a) establishing a flow of a purge gas immediately downstream of the catalyst,
b) contacting a pre-heated feed mixture comprising hydrogen, oxygen and a diluent with the catalyst at an elevated pressure, P2, which is less than P1, wherein the hydrogen to oxygen molar ratio is at least 0.5:1 and wherein the total feed mixture comprises less than 20wt% oxygen and at least 50wt% diluent,
c) increasing the oxygen concentration in the feed mixture until a catalyst exit temperature of at least 700°C is reached, and
d) increasing the pressure to the desired reaction pressure P1.

2. A method as claimed in claim 1, wherein the diluent in step (b) comprises the paraffinic hydrocarbon to be cracked.

3. A method as claimed in claim 1, wherein paraffinic hydrocarbon is not present in the pre-heated feed mixture in step (b) and is added either before or after the desired catalyst exit temperature is reached in step (c).

4. A method as in claim 1 or claim 2 wherein the hydrogen to oxygen molar ratio in the feed mixture to step (c) is maintained at at least 0.5:1 1 at least until combustion of paraffinic hydrocarbon is observed.

5. A method as claimed in any one of the preceding claims wherein prior to introduction of oxygen in step (b), the catalyst and reactor internals are pre-heated to a temperature of at least 150°C by passing a hot gas through the reactor and over the catalyst.

6. A method as claimed in claim 5 wherein the paraffinic hydrocarbon to be cracked is used as the hot gas.

7. A method as claimed in any one of the preceding claims wherein prior to introduction of oxygen in step (b), the oxygen system is continuously purged with inert gas.

8. A method as claimed in any one of the preceding claims wherein P2 is 5 to 15 barg.

9. A method as claimed in any one of the preceding claims wherein the oxygen is increased until the catalyst exit temperature is 750°C or above.

10. A method as claimed in any one of the preceding claims wherein the reaction is then held at reaction temperature after step (c) for an "activation" period of at least 15 minutes before step (d).

11. A method as claimed in any one of the preceding claims wherein in step (d) the pressure is increased by increasing flow rates with the pressure to maintain face velocity.

12. A method as claimed in any one of the preceding claims wherein a product stream quench is provided downstream of the catalyst exit, and wherein the quench is started prior to introduction of oxygen in step (b) of the present invention.
